# EUROPEAN PATENT APPLICATION

(11) **EP 1 449 504 A1**
(43) Date of publication of application: **25.08.2004**
(21) Application number: 04100686.7
(22) Date of filing: 20.02.2004
(51) Int. Cl.: A61F 9/04, A61F 11/12, A61F 9/02

(54) **Eye mask having receiving pockets for earplugs**

(30) Priority: 20.02.2003 NL 1022750
(71) Applicant: Nossbaum, Frederik Daniel, 2271 CA Voorburg (NL); Van Dorssen, Albert-Jaap, 2625 NS Delft (NL); Linders, Thomas Franciscus, 3021 AN Rotterdam (NL)
(72) Inventor: Nossbaum, Frederik Daniel, 2271 CA Voorburg (NL); Van Dorssen, Albert-Jaap, 2625 NS Delft (NL); Linders, Thomas Franciscus, 3021 AN Rotterdam (NL)
(74) Representative: Voncken, Bartholomeus Maria Christiaan

(57) **Abstract**

There is described a mask comprising a masking part covering the eyes of a user and attachment means connecting thereto. The mask is characterised by receiving pockets for removably housing therein earplugs. Preferably the earplugs are substantially concealed by the receiving pockets, and the earplugs are connected to the mask by means of a flexible means.

## Description

The invention relates to a mask comprising a masking part covering the eyes of a user and attachment means connecting thereto, characterised by receiving pockets for removably receiving earplugs.

Among others such masks are used on a large scale by airline passengers to get sleep during a flight without being disturbed by the cabin lights. However, such masks cannot prevent a user from being disturbed in its sleep by external noises.

US patent specification 2.942.270 already shows a mask comprising a cloth, the ends of which are shaped as earplugs.

Further, the state of the art discloses a number of publications, in which safety glasses are described with earplugs attached thereto. Examples thereof can be found, among others, in US patent specifications 6.340.227, 5.475.449, 3.943.925, 5.806.526, 5.541.677 and 6.082.855. With all these known arrangements it is a disadvantage that the earplugs, when not in use, can get entangled in external objects, and further are visible in a disturbing manner.

It is an object of the present invention to provide a mask of the type referred to above having an increased functionality.

In view of the above, in accordance with the present invention a mask is provided comprising a masking part covering the eyes of a user and attachment means connecting thereto, characterised by receiving pockets for removably receiving earplugs. When the mask is not being used or when the mask is only being used for covering the eyes without the necessity to conceal external noises, the earplugs may be positioned in the receiving pockets. In this position they do not in any way disturb the appearance of the mask, whereas further there is no risk that the earplugs get entangled in external objects. When the user desires to use the mask also for not being disturbed by external noises, the earplugs may be removed from the receiving pockets and may be positioned into the ears.

In a preferred embodiment of the mask in accordance with the invention, the receiving pockets fully conceal an earplug received therein. Like this the mask can be provided with a desired, aesthetically attractive appearance which is not disturbed by earplugs present therein.

In this respect it then is preferred, that the receiving pockets are positioned at the side of the masking part facing the face of a user. When the mask is present on the face of the user, the masking part fully conceals the receiving pockets.

Using a mask comprising material thickenings at the side of the masking part facing the face of a user for enhancing the fit of the mask to the face, it then is possible that the receiving pockets are positioned at interruptions in these material thickenings. As a result, by the application of the receiving pockets at the side of the masking part facing the face of the user the convenience for wearing it is not negatively influenced.

An alternative or additional manner for substantially fully concealing an earplug received in the receiving pockets, is when the receiving pockets define a practically cylindrical housing, for example oval-shaped, being substantially closed all around and comprising an access opening for the earplug.

Further a special embodiment of the mask is mentioned, wherein the receiving pockets are defined at cap-shaped clamping arrangements which are positioned substantially at the transitions between the masking part and the attachment means. For example, such cap-shaped clamping arrangements may be applied such as to attach an attachment strap to the masking part using the clamping action without the necessity of special attachment arrangements (such as sewing together the mentioned parts). Thus, the cap-shaped clamping arrangements obtain a dual function; connecting the attachment means and mask, and supporting the receiving pockets.

Although basically it is conceivable that the earplugs are fully detachable from the mask, it is also possible that the earplugs are connected to the mask by means of a flexible means, such as for example an elastic band or rope. Thus, it can be avoided that an earplug is lost during use or during storage. For this connection the cap-shaped clamping arrangement may be used too, wherein for example the flexible means is connected thereto in a manner avoiding tensile forces.

Hereinafter the invention will be elucidated referring to the drawing, in which an embodiment of the mask according to the invention is illustrated. Herein:
fig. 1 shows a partial view of an embodiment of the mask according to the invention from the side facing the face of a user;
fig. 2 shows the two constituting parts of a cap-shaped clamping arrangement used in fig. 1, and
fig. 3 shows a cross-section according to III-III in fig. 1.

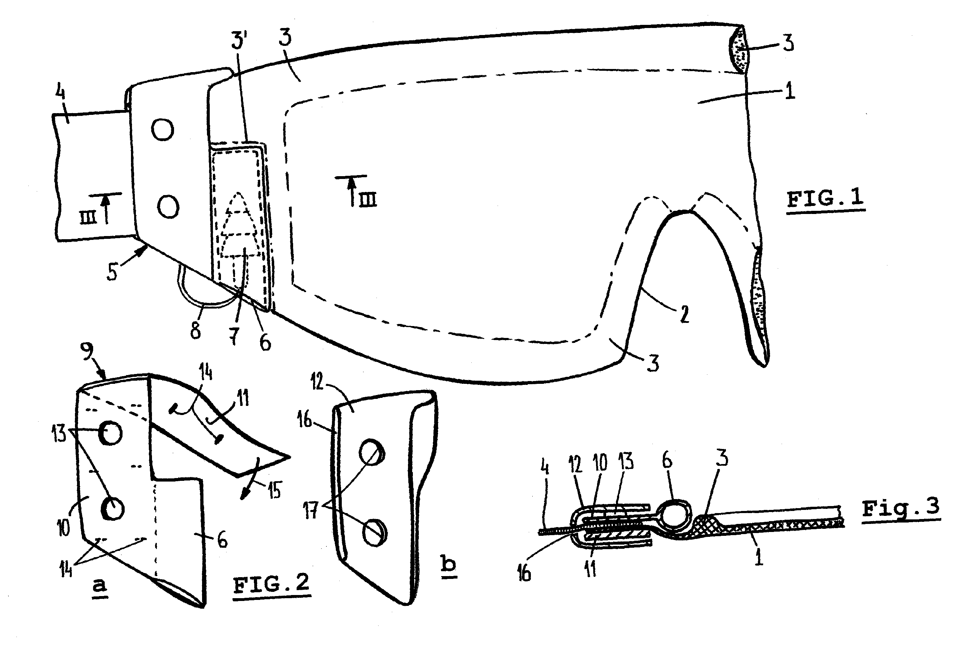

In fig. 1 part of a mask is illustrated, seen from the side facing the face of a user. The mask comprises a masking part 1 for covering the eyes of a user. In the masking part 1 a nose recess 2 is shaped in a conventional manner. At appropriate locations material thickenings 3 are shaped on the masking part 1 which provide an optimal fit to the face of the user and which maintain the mask with its larger part at a distance form the face, thus at one hand avoiding an unpleasant pressure on the eyes and at the other hand avoiding sweating due to a contact.

Further, the mask is provided with an attachment strap 4 which can be connected to the masking part 1 in any appropriate manner. This attachment strap 4 may comprise one part which with its both ends is connected to the masking part 1; however, it is possible too that the attachment strap 4 comprises two parts which may be interconnected in a settable manner or not. Different attachment means are conceivable too as an alternative to the attachment strap, such as legs or a cord.

In the illustrated embodiment of the mask according to the invention a cap-shaped clamping arrangement 5 is provided at the transition between the masking part 1 and the attachment strap 4. It will be elucidated below while referring to fig. 2 and 3. The cap-shaped clamping arrangement 5 is provided with a receiving pocket 6 connected thereto for an earplug 7. In the illustrated embodiment the earplug 7 is connected to the cap-shaped clamping arrangement 5 in a manner avoiding tensile forces, by means of a flexible means too, for example an elastic band 8. The earplug 7 is removably positioned in the receiving pocket 6 and may be removed therefrom at will by a user for being positioned into an ear.

The receiving pocket 6 can assume several shapes. For example this receiving pocket 6 may define an oval-shaped housing which is substantially closed all around and comprises an access opening for the earplug 7. Like this an earplug 7 positioned in the receiving pocket 6 is concealed.

In the illustrated embodiment the receiving pocket 6 is positioned at the side of the masking part 1 facing the face of a user. Further the receiving pocket 6 is positioned at an interruption 3' in the material thickening 3.

Now, the cap-shaped clamping arrangement 5 will be elucidated while referring to fig. 2 and 3. As illustrated in fig. 2, the cap-shaped clamping arrangement 5 comprises two parts: a clamping part 9 comprising to pivotably connected wings 10 and 11 (fig. 2a) and a clamping cap 12 (fig. 2b). One of the wings 10 of the clamping part 9 supports the receiving pocket 6 for the earplug, and comprises two notches 13. At their sides facing towards each other in the operative position, wings 10 and 11 comprise projections 14. At the junction between the attachment strap 4 and the masking part 1 the clamping part 9 will be positioned, while the wings 10 and 11 are pivoted towards each other (in correspondence with arrow 15). Here, the projections 14 engage the material of the attachment strap 4 and the masking part 1 in a locking manner. Next the clamping cap 12 is slid over the attachment strap 4 (see figures 1 and 3) whereby this attachment strap 4 is led through a recess 16 of the clamping cap, until the clamping cap 12 is positioned over de clamping part 9. In this position the notches 13 project through corresponding recesses 17 provided in the clamping cap 12. As a result, the mutual position of clamping cap 12 and clamping part 9 is locked. In this position the clamping part 9 firmly engages the masking part 1 and the attachment strap 4, and the position thereof is fixed. For disengaging the mentioned part a reversed order is followed.

Figure 3 schematically illustrates a cross section according to line III-III in fig. 1. It shows the masking part 1 with material thickening 3, wing 11 of the clamping part 9 and the other wing 10 thereof with receiving pocket 6 and notch 13, and finally the clamping cap 12 with opening 16.

The invention is not limited to the embodiment described above, which may be varied widely within the scope of the invention as defined by the claims. Thus, the receiving pockets also may be positioned at the side of the mask facing away from the face.

## Claims

1. Mask comprising a masking part covering the eyes of a user and attachment means connecting thereto, **characterised by** receiving pockets for removably receiving earplugs.

2. Mask according to claim 1, wherein the receiving pockets are shaped such as to substantially conceal an earplug received therein.

3. Mask according to claim 1 or 2, wherein the receiving pockets are positioned at the side of the masking part facing the face of a user.

4. Mask according to claim 3, comprising material thickenings at the side of the masking part facing the face of a user enhancing the fit of the mask to the face, wherein the receiving pockets are positioned at interruptions in these material thickenings.

5. Mask according to one of the claims 2-4, wherein the receiving pockets define a practically cylindrical housing being substantially closed all around and comprising an access opening for the earplug.

6. Mask according to one of the previous claims, wherein the receiving pockets are defined at cap-shaped clamping arrangements which are positioned substantially at the transitions between the masking part and the attachment means.

7. Mask according to one of the previous claims, wherein the earplugs are attached to the mask by means of a flexible means, such as for example an elastic band or rope.
